## Europäisches Patentamt
## European Patent Office
## Office européen des brevets

⑩

(11) Publication number: **0 109 091**
**B1**

(12) **EUROPEAN PATENT SPECIFICATION**

(45) Date of publication of patent specification: **16.08.90**

(21) Application number: **83111448.3**

(22) Date of filing: **15.11.83**

(51) Int. Cl.⁵: **G 01 T 1/164, A 61 B 6/00**

(54) Scintillation camera apparatus.

(30) Priority: **16.11.82 JP 199745/82**

(43) Date of publication of application:
**23.05.84 Bulletin 84/21**

(45) Publication of the grant of the patent:
**16.08.90 Bulletin 90/33**

(84) Designated Contracting States:
**DE GB NL**

(56) References cited:
**EP-A-0 066 917**
**GB-A- 15 471**
**US-A-3 892 967**
**US-A-4 216 381**

(73) Proprietor: **KABUSHIKI KAISHA TOSHIBA**
**72, Horikawa-cho Saiwai-ku**
**Kawasaki-shi Kanagawa-ken 210 (JP)**

(72) Inventor: **Iwakoshi, Keiichi**
**2-7-20, Shintomi-cho**
**Ootawara-shi Tochigi-ken (JP)**
Inventor: **Tsutsumi, Masaaki**
**526-4, Iguchi Nishinasuno-machi**
**Nasu-gun Tochigi-ken (JP)**

(74) Representative: **Henkel, Feiler, Hänzel & Partner**
**Möhlstrasse 37**
**D-8000 München 80 (DE)**

Courier Press, Leamington Spa, England.

## Description

The present invention relates to a scintillation camera apparatus which has a scintillation camera which detects, radioisotope (to be referred to as "RI" hereinafter) distribution data of an object to be examined, e.g., a patient who has been administered an RI, from various angles with respect to the axis of the object and which provides tomographic images based on the RI distribution data. More specifically, the present invention relates to a scintillation camera apparatus which supports an RI detector in accordance with the principle of weight-balance as defined in claims 1, 3 and 5.

A scintillation camera captures gamma rays, for example, radiated from the RI administered in a patient. A brightness spot is displayed at a position on a cathode ray tube which corresponds to the position of the detector on which the radiated gamma rays are incident. The camera then grasps the RI distribution (referred to as a scintigram) within a body of the patient so as to allow diagnosis of a position of a diseased portion, of a spread condition of a diseased portion or the like.

An Emission Computed Tomography apparatus (to be referred to as an "ECT" apparatus) has recently been developed and receiving a lot of attention. The ECT apparatus produces tomographs of a patient using a scintillation camera as described above. The ECT apparatus uses, for example, an anger scintillation camera as a radiation detector. The scintillation camera is rotated stepwise or continuously around the object who has been administered the RI. Gamma rays emitted from the RI in the patient are detected at various angles with respect to the axis of the patient. The obtained detection data is processed to reconstruct tomographs of the patient. In an apparatus having such an ECT function, a detector ordinarily weighting about 600 to 800 kg must be supported such that it can rotate around the longitudinal axis of the patient through 360° and it can approach to or depart from the patient. Various detector support mechanisms for supporting such a detector have been devised.

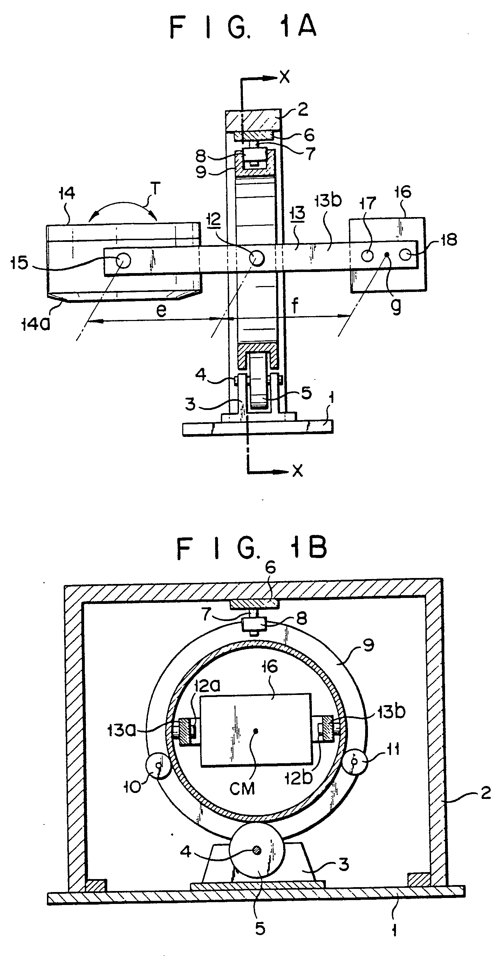

The ECT apparatus will now be described with reference to an apparatus having an ECT function and incorporating a supporting mechanism for supporting a conventional RI detector, as shown in Figs. 1A and 1B (a longitudinal sectional view taken along the line X—X in Fig. 1A). A support frame 2 and a roller support member 3 are securely fixed on a base 1 fixed on a floor (not shown). In the roller support member 3, a support roller 5 is pivotally supported by a shaft 4. A roller support base 6 is fixed at the center of the upper portion of the support frame 2, and a guide roller 8 is rotatably supported by a shaft 7 extending downward from the base 6. A circular ring member 9 is rotatably supported by the support roller 5 and a guide roller 8. Thus, the support roller 5 supports the weight of the circular ring member 9, and the guide roller 8 guides the circular ring member 9 such that the member 9 will not be inclined in the clockwise direction with respect to a horizontal plane in Fig. 1A. The apparatus further has two supplementary rollers 10 and 11 for supplementarily supporting the rotating circular ring member 9, thereby allowing smooth rotation of the circular ring member 9 in the clockwise and counterclockwise directions in a plane of the drawing of Fig. 1B. Two beams 13a and 13b are pivotally supported by shafts 12a and 12b, respectively, so as to extend at two sides of and inside the circular ring member 9 and to extend therethrough. An RI detector 14 for detecting radiation from a patient (not shown) is supported by one end (to the left of the support frame 2 in Fig. 1A) of each of the beams 13a and 13b through two shafts 15 (only one shown in the figure) so as to be pivotable in a direction indicated by arrow "T". The RI detector 14 is generally constituted by the collimator, the scintillator, the light guide and the photomultiplier tube which are optically aligned along the incident light path. Accordingly this RI detector 14 is also referred to as a scintillation camera means. A counterweight 16 having a weight given by equation (1) below is fixed by two fixing shafts 17 and 18 at the other end of each of the beams 13a and 13b.

$$W1 = W2 \cdot f/e \qquad (1)$$

where
    W1: the weight of the RI detector 14
    W2: the weight of the counterweight 16
    e: the distance between the shafts 12 and 15
    f: the distance between the shaft 12 and a center of gravity "g" of the counterweight 16.

To obtain tomographs of a patient using a conventional ECT apparatus having a construction as described above, the beams 13a and 13b are inclined by a predetermined angle. The patient is layed down along a rotating shaft of the circular ring member 9, that is, along an axis horizontally extending across a center of rotation $C_M$. The detector 14 is rotated about the shaft 15 and prepared such that a detection surface 14a thereof faces the patient. Thereafter, the circular ring member 9 is rotated through 360° at a pitch of 60° in a clockwise direction with respect to an axis of rotation "$C_M$" shown in Fig. 1B and detection data of gamma rays at each angle are collected so as to reconstruct tomographs. Similar techniques are also described in document US—A—4,216,381.

However, in a conventional ETC apparatus as described above, if the distance "e" must exceed a predetermined distance, the following requirement must be satisfied:

(1) the distance "f" is set to be long in accordance with the equation (1) above; or

(2) the weight "W2" of the counterweight 16 is increased in accordance with the equation (1) above. This results in problems to be discussed below.

More specifically, when the requirement of the item (1) is to be satisfied, the length of the beams 13a and 13b extending from the circular ring member 9 is increased. This causes a bulky ECT apparatus. When the requirement of item (2) is to be satisfied, the length of the beams 13a and 13b extending from the member 9 can be reduced. However, the weight of the counterweight 16 must be increased. This results in a heavier ECT apparatus as a whole. In view of these problems, a method has been generally adopted in an ECT apparatus in which the weight of the detector 14 is set to equal that of the counterweight 16 and the distance "e" is set to equal the distance "f", in consideration of the requirements of the items (1) and (2).

Furthermore, in the conventional ECT apparatus as described above, the detector 14 is supported by the beams 13a and 13b to be rotatable about the shaft 15. When the beams 13a and 13b are rotated clockwise about the shafts 12a and 12b, the angle of the detection surface 14a of the detector 14 with respect to the floor surface (horizontal plane) also changes. This is inconvenient in diagnosis (tomographic image reconstruction of the entire body of the patient or ECT photography) in which the detection surface 14a must be kept at a constant angle with respect to the patient (layed down parallel to the horizontal plane). This gives rise to a need for a rotational angle adjustment such that the detection surface 14a is kept at a constant angle from the patient.

Prior art document EP—A—0 066 917 discloses a radiation apparatus comprising a camera which is to be positioned with respect to an object and which is carried by a balanced suspension system having a counterweight. The suspension system has a parallelogram structure from opposite ends of which the camera to be positioned and the counterweight are suspended via pivotal supports so as to balance one another. One of the pivotal supports comprises a non-supporting pivot for an arm for positioning the camera to be positioned, and a supporting pivot for the counterweight. The pivotal supports are mounted in a supporting ring which is rotatable about an isocentric axis of the object, and the pivot which supports the counterweight is situated on the opposite side of the isocentric axis to the camera to be positioned.

It is an object of the present invention to provide a compact and lightweight scintillation camera apparatus which is free from the problems of conventional apparatus, and which does not require complex or time-consuming procedures such as adjustment of the respective components prior to or during diagnosis.

Those objects will be accomplished by providing a scintillation camera apparatus as defined in any one of claims 1, 3 and 5.

For a better understanding of these and other objects of the present invention, reference is had to the following detailed description of the invention to be read in conjunction with the following drawings, in which:

Fig. 1A is a cross-sectional view of the conventional scintillation camera apparatus;

Fig. 1B is a longitudinal sectional view taken along the line X—X in Fig. 1A;

Fig. 2A is a cross-sectional view of a scintillation camera apparatus according to one preferred embodiment of the invention;

Fig. 2B is a longitudinal sectional view taken along the line Y—Y in Fig. 2A;

Fig. 3 is a schematic illustration for explaining principle of the weight-balance employed in the present invention;

Fig. 4 is a cross-sectional view of a scintillation camera apparatus according to another embodiment; and

Fig. 5 is a cross-sectional view of a scintillation camera apparatus according to a further embodiment.

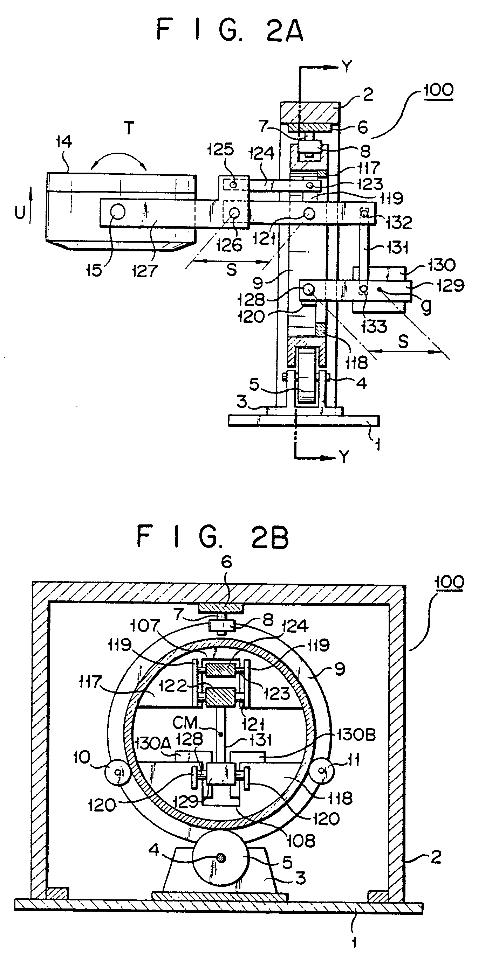

A scintillation camera apparatus 100 according to an embodiment of the present invention will now be described with reference to Figs. 2A and 2B taking account of the conventional apparatus as described above. The same reference numerals as in Figs. 1A and 1B denote the same parts in Figs. 2A and 2B, and a detailed description thereof will be omitted. Reference numeral 1 denotes the base as a support member which is fixed on a floor (not shown). In other words, the base 1 is positioned to be extended in a first plane perpendicular to the floor plane. A support frame 2 and a roller support member 3 are securely fixed on the base 1. A shaft 4 rotatably supports a support roller 5. A roller support base 6 rotatably supports a guide roller 8 by means of a fixed shaft 7. A circular ring member 9 is rotatably supported in a second plane perpendicular to the floor plane as well as the first plane by the support roller 5, the guide roller 8 and two supplementary rollers 10 and 11.

In the scintillation camera apparatus 100 having the construction as described above, a structure to be described below is coupled to the constituent parts of the apparatus as shown in Figs. 1A and 1B.

In the state shown in Figs. 2A and 2B (a longitudinal sectional view taken along the line Y—Y in Fig. 2A), an upper supporting base plate 117 and a lower supporting base plate 118 are arranged at the inner upper and lower sides of the circular ring member 9. The base plates 117 and 118 have opposing notch members 107 and 108, respectively, which are notched at their centers in U-shapes. A pair of guide plates 119 are arranged at the two sides of the notch member 107, while a pair of guide plates 120 are similarly arranged at the two sides of the notch member 108. A first beam supporting shaft 121 is suspended between the pair of guide plates 119 of the upper supporting base plate 117. The first beam supporting shaft 121 rotatably supports a first beam 122. A second beam supporting shaft 123 is suspended between the pair of guide plates 119 above the first beam supporting shaft 121 so as to rotatably support a second beam 124. An L-shaped detector supporting arm 127 is pivotally supported at one end of each of the first and second beams 122 and 124 through two pivot shafts 125 and 126, respectively. The shaft 15 arranged at the ends of the detector supporting arm 127 support an RI detector 14 such that the detector 14 is rotatable in a direction indicated by arrow T. In other words, the detector 14 is supported by parallel link mechanisms consisting of the shafts 121, 123, 125 and 126 and the beams 122 and 124. An arm supporting shaft 128 is suspended between the pair of guide plates 120 fixed to the lower supporting base plate 118. The arm supporting shaft 128 rotatably supports one end of an arm or a third beam 129. Two counterweights 130A and 130B which constitute one counterweight 130 are fixed to the other end of the arm 129. A rod 131 is suspended between the arm 129 and the first beam 122 such that the two ends of the rod 131 are pivotally supported by rod shafts 132 and 133, respectively. In this case, the distance between the first beam supporting shaft 121 and the rod shaft 132 is set to be equal to that between the arm supporting shaft 128 and the rod shaft 133.

The mode of operation for taking tomographs by the apparatus having the construction as described above will now be described with reference to Figs. 2A and 2B.

First, the detector 14 is moved upward in a direction indicated by arrow "U" from the state shown in Fig. 2A. A patient (not shown) who has been administered an RI is layed down on a plane crossing an axis of center of rotation (to be described later) of the circular ring member 9. Then the detector 14 is located at an optimal position separated from the patient. Rotation of the detector 14 in a direction indicated by arrow "T" and movement thereof in a direction indicated by arrow "U" are locked by an electromagnetic locking mechanism (not shown) so as to set the detector 14. Thereafter, the circular ring member 9 is rotated clockwise, for example, in Fig. 2B through a predetermined angle or 360° by a motor (not shown) or the like so as to detect radiated gamma rays from the patient at various angles. The obtained data is stored. After all the required data has been collected, a tomograph is reconstructed by computer-processing the obtained data.

According to the scintillation camera apparatus of the present invention, the advantage of principle of weight-balance method is obtained. More specifically, if the weights of the detector and the counterweight, and their distances from the rotating shaft satisfy a predetermined relationship, a balance can be established irrespective of the rotating angle of the circular ring member and the inclined angles of the beams. In addition, the length of the counterweight extending from the supporting frame can be reduced, and the detection surface of the detector does not change in position with respect to the inclined angles of the beams. This will be described in more detail hereinafter.

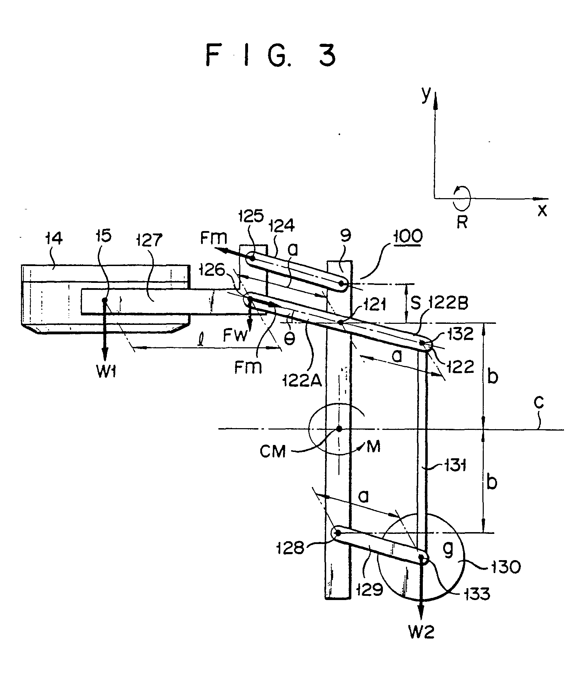

Fig. 3 is a representation showing the balance between the detector 14 and the counterweight 16. The following conditions are assumed with the apparatus having the construction as described above.

(1) The weight (W1) of the detector 14 is equal to that (W2) of the counterweight 16. (W1=W2).

(2) A first half beam portion 122A of the first beam 122 and the second beam 124 constitute a first parallel link mechanism.

(3) A second half beam portion 122B of the first beam 122 and the arm 129 constitute a second parallel link mechanism. The first beam 122 consists of the first and second half beam portions 122A and 122B. The first half beam portion 122A partially constitutes the first parallel link mechanism while the second half beam portion 122B partially constitutes the second parallel link mechanism.

(4) The rod 131 and the circular ring member 9 hold a parallel relation.

(5) The distance between the first beam supporting shaft 121 and the pivot shaft 126, that is, a working length "a" of the first half beam portion 122A is equal to the distance between the arm supporting shaft 128 and the center of gravity "g" of the counterweight 130, that is, a working length "a" of a third beam 129.

(6) The distance "b" between the first beam supporting shaft 121 and the axis of rotation "$C_M$" of the circular ring member 9 is equal to that "b" between the arm supporting shaft 128 and the axis of rotation "$C_M$" of the circular ring member 9.

The above conditions presuppose the following conditions as well.

(a) The positions of the detector 14 and the counterweight 130 are at the material points.

(b) The weights of the arms, links, etc. are considered to be negligible.

A reference plane is considered before explaining the balance operation.

A first plane is defined to be perpendicular to the floor plane (not shown). The base 1 is fixed to the floor plane to extend along the first plane. The first plane is parallel to the plane shown in Fig. 2B. In other words, the first plane is perpendicular to the plane of the sheet of the drawing of Fig. 2A and extends along the line Y—Y thereof. The circular ring member 9 as a rotating member is supported by the rollers 5, 8, 10 and 11 so as to rotate clockwise or counterclockwise about the center of rotation "$C_M$" within the first plane.

A second plane is assumed which is perpendicular to both the first plane and to the floor plane. In other words, the second plane is parallel to the plane of the sheet of the drawing of Figs. 2A and 3. Two parallel link mechanisms are constituted in the second plane. The first half beam portion 122A of the first beam 122 and the second beam 124 constitute the first parallel link mechanism under the condition of item (2) above. One end of the first parallel link mechanism is pivotally journalled to the circular ring member 9 through the supporting shafts 121 and 123. The other end of the first parallel link mechanism is pivotally journalled by the L-shaped detector supporting arm 127 through the pivot shafts 126 and 125.

Meanwhile, the second half beam portion 122B of the first beam 122 and the third beam 129 constitute the second parallel link mechanism under the condition of item (3) above. One end of the third beam 129 is pivotally journalled by the circular ring member 9 through the arm supporting shaft 128. As has been described earlier, one end of the second half beam portion 122B is pivotally supported by the circular ring member 9 through the shaft 121. The other ends of the beams 122B and 129 are pivotally supported by the connection rod 131 through the rod shafts 132 and 133. The counterweight 130 is fixed to the other end of the third beam 129.

When a correspondence of the planes with the x-y-z plane as shown in Fig. 3 is considered, the floor plane corresponds to the x-z plane, the first plane corresponds to the y-z plane, and the second plane corresponds to the x-y plane, respectively. The circular ring member 9 rotates about the axis of rotation "$C_M$" within the y-z plane.

The weight W1 of the detector 14 produces a force of moment "Fm" and a moment "Fw" by the weight of the detector, which are given below, on the two pivot shafts 125 and 126 of the collimator supporting arm.

$$Fm = W1 \cdot l/(S \cdot \cos\theta), \quad W1 = Fw$$

where

l: the distance between the shaft 15 and the pivot shaft 126;

S: the distance between the first and second beam supporting shafts 121 and 123; and

θ: the inclined angle of the first beam supporting shaft 121 with respect to a line of rotation center "C".

This moment component generates a moment "M" ($M = W1 \cdot l$) in the circular ring member 9 through the parallel link mechanism consisting of the first and second beams 122 and 124. The moment "M" is absorbed by the roller 8 supporting the circular ring member 9 and does not affect the balance between the detector 14 and the counterweight 16. Accordingly, only the moment "Fw" is related to the balance between the detector 14 and the counterweight 16.

The moment "M" described above affects the balance in the x direction (that is, in the counterclockwise direction indicated by the x-y plane in Fig. 3). However, as described above, this moment "M" is absorbed by the roller 8 supporting the circular ring member 9 and therefore does not disturb the balance.

The balance in the y direction (in the direction perpendicular to the x direction) is given by:

$$Fw \times a \cdot \cos\theta = W2 \times a \cdot \cos\theta, \quad Fw = W2$$

where

W2 is the weight of the counterweight 130, thus providing a good balance.

When the balance in the "R" direction (around the line of rotation center "C" parallel to the x-axis) is considered, it is proved by showing the balance between the detector 14 and the counterweight 130 when the direction in which gravity acts is set to be perpendicular to the plane of the sheet of the drawing of Fig. 3. Thus, as in the case of the balance in the x direction, we obtain:

$$Fw \times (b + a \cdot \sin\theta) = W1 \times (b + a \cdot \sin\theta)$$

where

'b' is the distance between the beam supporting shafts 121 and 128 and the center of rotation "$C_M$". Therefore, Fw = W1 and a good balance is obtained.

In the present invention wherein the detector 14 is supported with good balance in this manner, the length of the counterweight 130 extending outward from the support frame 2 is reduced without requiring an increase in the weight of the counterweight 130. Accordingly, the detector 14 and the counterweight 130 are kept balanced independently of the rotational angle of the circular ring member 9 and the inclined angles of the first and second beams 122 and 124. Thus, the apparatus can be rendered compact in size and light in weight. As another feature of the apparatus of this type, the distance "l" between the pivot shaft 126 and the center of gravity of the detector 14 is independent of the balance between the detector 14 and the counterweight 130. Therefore, the distance "l" can be freely set unlike the distance "a" between the arm supporting shaft 128 and the center of gravity "g" of the counterweight 130.

The present invention is not limited to the particular embodiment described above. For example, the above embodiment is described with reference to a scintillation camera apparatus having an ECT function. However, the present invention can be similarly applied to other types of scintillation camera apparatus.

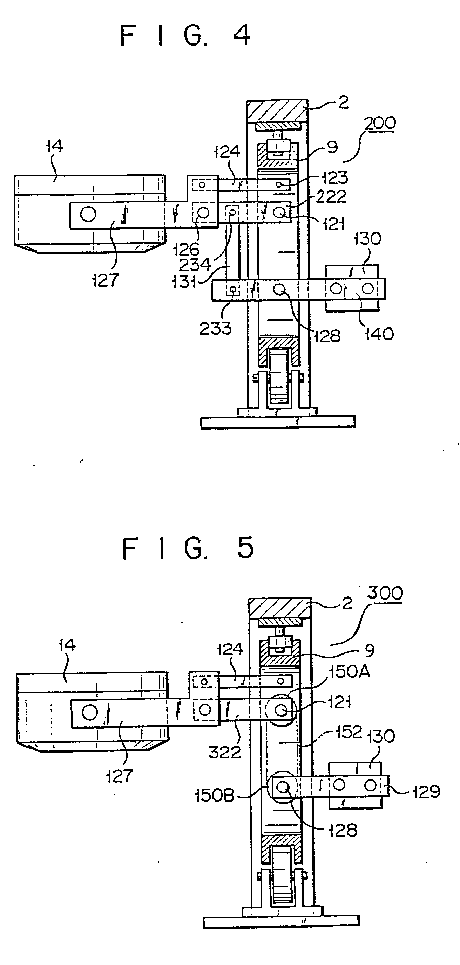

An apparatus 200 as shown in Fig. 4 can be adopted. In the apparatus 200 shown in Fig. 4, an arm or a third beam 140 corresponding to the arm 129 as shown in Fig. 2 projects toward the detector 14 as well. A rod 131 couples an end of the arm 140 with a first beam 222. The first beam 222 has a working length "a" which is the same as that of the first half beam portion 122A shown in Fig. 2. A pivot shaft 234 on which the connection rod 131 is pivotally mounted is interposed between the shafts 121 and 126. A pivot shaft 233 for pivotally supporting the connection rod 131 is arranged at the end of the third beam 140 which is at the side of the detector 14. In this case, the balance is obtained by the same principle as in the case described with reference to the equations of the balance in the embodiment described above. Another apparatus 300 as shown in Fig. 5 can also be adopted. Referring to Fig. 5, a sprocket 150A is fixed to a first beam supporting shaft 121 of a first beam 322, and another sprocket 150B is fixed to an arm supporting shaft 128 of a third beam 129. A chain 152 connects these sprockets 150A and 150B. In this case, the balance is different from that described with reference to the above embodiment. However, if the gear ratio of the sprockets 150A and 150B is set to be 1:1, a good balance between the detector 14 and the counterweight 130 can be established. The same reference numerals as in Fig. 2 denote the same parts in Figs. 4 and 5.

In summary, there is provided according to the present invention a scintillation camera apparatus which is compact in size and which has excellent operability in various diagnosis modes.

**Claims**

1. A scintillation camera apparatus (100) comprising:

support means (2) which is fixed on the plane of a floor, and extends in a first plane perpendicular to said floor plane;

rotation means (9) which has a rotation axis (CM) perpendicular to said first plane, said rotation means being rotatably secured to the support means (2) in said first plane;

first parallel link means comprising:

parallel members (122A, 124) pivotally journalled at their first ends to the rotation means (9), so as to be pivotable in a second plane perpendicular to said first plane, and at their second ends to first connection means (127), one end of which is pivotally journalled to said parallel members (122A, 124) and the other end of which is pivotally journalled to scintillation camera means (14);

second parallel link means comprising:

parallel members (122B, 129) pivotally journalled at their first ends to the rotation means (9) so as to be pivotable in said second plane, and at their second ends to second connection means (131), said members (122B, 129) extending from the opposite side of rotation means (9) to the first parallel link means;

one member of the first parallel link means and one member of the second parallel link means having a common attachment point to the rotation means (9) and being formed by one common member (122), the attachment point for said common member (122) being located between the attachment points for the other members of the first and second parallel link means;

counterweight means (130) attached to said second connection means.

2. A scintillation camera apparatus (100) as claimed in claim 1, wherein:

said rotation means (9) is a circular ring member which is pivotally journalled to support means (2) through a plurality of rollers (5, 8, 10, 11);

said first parallel link means is formed by a first half-beam portion (122A) of a first beam (122), and a second beam (124) of equal working lengths a;

said second parallel link means is formed by a second half-beam portion (122B) of said first beam (122) and a third beam (129) of equal working lengths a;

said first beam (122) is pivotally journalled via a first beam rotation shaft (121) to said rotation means (9), and said third beam (129) is pivotally journalled via third beam rotation shaft (128) to said rotation means (9);

the distance (b) between the first beam rotation shaft (121) and the rotation axis (CM) is equal to the distance (b) between the third beam rotation shaft (128) and the rotation axis (CM) and;

the weight of the counterweight (130) is identical to that of said scintillation camera means (14).

3. A scintillation camera apparatus (200) comprising:

support means (2) which is fixed on the plane of a floor, and extends in a first plane perpendicular to said floor plane;

rotation means (9) which has a rotation axis (CM) perpendicular to said first plane, said rotation means being rotatably secured to the support member (2) in said first plane;

first parallel link means comprising:

parallel members (222, 124) pivotally journalled at their first ends to the rotation means (9), so as to be pivotable in a second plane perpendicular to said first plane, and at their second ends to first connection means (127), one end of which is pivotally journalled to said parallel members (222, 124) and the other end of which is pivotally journalled to scintillation camera means (14);

second parallel link means formed by:

part of one of the members (222) of said first parallel link means and part of a further member (140), pivotally journalled a point between its ends to the rotation means (9) so as to be pivotable in said second plane, a first end of said further member, located on the same side of rotation means (9) as the first parallel link means, being pivotally journalled to one end of second connection means (131);

second connection means (131) pivotally journalled at one end to said first end of said further member, and at the other end to a point of said one of the members (222) of said first parallel link means located between its ends;

counterweight means (130) attached to the second end of said further member.

4. A scintillation camera apparatus (200) as claimed in claim 3, wherein:

said rotation means (9) is a circular ring member which is pivotally journalled to support means (2) through a plurality of rollers (5, 8, 10, 11);

said first parallel link means is formed by a first beam (222) and a second beam (124) of equal working lengths a;

said second parallel link means is formed by part of said first beam (222) and a part of third beam (140), the working length of the other part of third beam (140) being a;

said first beam (222) is pivotally journalled via a first beam rotation shaft (121) to said rotation means (9) and said third beam (140) is pivotally journalled via a third beam rotation shaft (128) to said rotation means (9);

the distance (b) between the first beam rotation shaft (121) and the rotation axis (CM) is equal to the distance (b) between the third beam rotation shaft (128) and the rotation axis (CM) and;

the weight of the counterweight (130) is identical to that of said scintillation camera means (14).

5. A scintillation camera apparatus (300) comprising:

support means (2) which is fixed on the plane of a floor, and extends in a first plane perpendicular to said floor plane;

rotation means (9) which has a rotation axis (CM) perpendiclar to said first plane, said rotation means being rotatably secured to the support means (2) in said first plane;

parallel link means comprising:

parallel members (322, 124) pivotally journalled at their first ends to the rotation means (9), so as to be pivotable in a second plane perpendicular to said first plane, and at their second ends to first connection means (127), said link means being formed by a first beam (322) and a second beam (124) of equal working lengths a, said first beam (322) being pivotally journalled via a first beam rotation shaft (121) to the rotation means (9);

first connection means (127), one end of which is pivotally journalled to said parallel members (222, 124) and the other end of which is pivotally journalled to scintillation camera means (14);

second link means comprising:

a third beam (129) of working length a, pivotally journalled to the rotation means (9) so as to be pivotable in said second plane, one end of said third beam (129) being pivotally journalled via a third beam rotation shaft (128) to the rotation means (9), and counterweight means (130) being rigidly connected to the other end thereof;

second connection means comprising a pair of sprocket wheels (150A, 150B), one of said sprocket wheels (150A) being rotatably connected to the first beam rotation shaft (121) and fixed to said first beam, and the other of said sprocket wheels (150B) being rotatably connected to the third beam rotation shaft (128) and fixed to said third beam, and a chain (152) connecting the sprocket wheels (150A, 150B).

## Patentansprüche

1. Szintillationskamerägerät (100) mit:

einer Trägereinrichtung (2), die auf der Ebene eines Bodens festgelegt ist und sich in einer ersten Ebene senkrecht zur Bodenebene erstreckt;

einer Rotationseinrichtung (9), die eine Rotationsachse (CM) senkrecht zur ersten Ebene hat, wobei die Rotationseinrichtung drehbar an der Trägereinrichtung (2) in der ersten Ebene befestigt ist;

einer ersten Parallelgelenkeinrichtung mit:

parallelen Gliedern (122A, 124), die schwenkbar an ihren ersten Enden an der Rotationseinrichtung (9) angelenkt sind, um schwenkbar in einer zweiten Ebene senkrecht zur ersten Ebene zu sein, und die an ihren zweiten Enden schwenkbar an einer ersten Verbindungseinrichtung (127) angelenkt sind, von der ein Ende schwenkbar an den parallelen Gliedern (122A, 124) schwenkbar angelegt ist und von der das andere Ende schwenkbar an einer Szintillationskamereinrichtung (14) angelenkt ist;

einer zweiten Parallelgelenkeinrichtung mit:

parallelen Gliedern (122B, 129), die schwenkbar an ihren ersten Enden an der Rotationseinrichtung (9) angelenkt sind, um schwenkbar in der zweiten Ebene zu sein, und die an ihren zweiten Enden schwenkbar an einer zweiten Verbindungseinrichtung (131) angelenkt sind, wobei sich diese Glieder (122B, 129) von der entgegengesetzten Seite der Rotationseinrichtung (9) zu der ersten Parallelgelenkeinrichtung erstrecken; wobei ein Glied der ersten Parallelgelenkeinrichtung und ein Glied der zweiten Parallelgelenkeinrichtung einen gemeinsamen Befestigungspunkt an der Rotationseinrichtung (9) haben und aus einem gemeinsamen Glied (122) bestehen, wobei der Befestigungspunkt für das gemeinsame Glied (122) zwischen den Befestigungspunkten für die anderen Glieder der ersten und zweiten Parallelgelenkeinrichtungen liegt;

einer Gegengewichteinrichtung (130), die an der zweiten Verbindungseinrichtung angebracht ist.

2. Szintillationskameragerät (100) nach Anspruch 1, bei dem:

die Rotationseinrichtung (9) ein kreisförmiges Ringglied ist, das drehbar an der Trägereinrichtung (2) über eine Vielzahl von Rollen (5, 8, 10, 11) angebracht ist;

die erste Parallelgelenkeinrichtung aus einem ersten Halbbalkenteil (122A) eines ersten Balkens (122) und einem zweiten Balken (124) von gleichen Arbeitslängen a besteht;

die zweite Parallelgelenkeinrichtung aus einem zweiten Halbbalkenteil (122B) des ersten Balkens (122) und einem dritten Balken (129) von gleichen Arbeitslängen a besteht;

der erste Balken (122) schwenkbar über eine erste Balkenrotationsachse (121) an der Rotationseinrichtung (9) angelenkt ist und der dritte Balken (129) schwenkbar über eine dritte Balkenrotationsachse (128) an der Rotationseinrichtung (9) angebracht ist;

der Abstand (b) zwischen der ersten Balkenrotationsachse (121) und der Rotationsachse (CM) gleich dem Abstand (b) zwischen der dritten Balkenrotationsachse (128) und der Rotationsachse (CM) ist; und

das Gewicht des Gegengewichtes (130) identisch zu demjenigen der Szintillationskameraeinrichtung (14) ist.

3. Szintillationskameragerät (200) mit:

einer Trägereinrichtung (2), die auf der Ebene eines Bodens festgelegt ist und sich in einer ersten Ebene senkrecht zur Bodenebene erstreckt;

einer Rotationseinrichtung (9), die eine Rotationsachse (CM) senkrecht zur ersten Ebene hat, wobei die Rotationseinrichtung drehbar an der Trägereinrichtung (2) in der ersten Ebene befestigt ist;

einer ersten Parallelgelenkeinrichtung mit:

parallelen Gliedern (222, 124), die schwenkbar an ihren ersten Enden an der Rotationseinrichtung (9) angelenkt sind, um schwenkbar in einer zweiten Ebene senkrecht zur ersten Ebene zu sein, und die an ihren zweiten Enden schwenkbar an einer ersten Verbindungseinrichtung (127) angelenkt sind, von der ein Ende schwenkbar an den parallelen Gliedern (222, 124) angelenkt ist und das andere Ende Ende schwenkbar an einer Szintillationskamerameinrichtung (14) angelenkt ist;

einer zweiten Parallelgelenkeinrichtung aus:

einem Teil von einem Glied der Glieder (222) der ersten Parallelgelenkeinrichtung und einem Teil eines weiteren Gliedes (140), das schwenkbar an einem Punkt zwischen seinen Enden an der Rotationseinrichtung (9) angelenkt ist, um in der zweiten Ebene schwenkbar zu sein, wobei ein erstes Ende des weiteren Gliedes, auf der gleichen Seite der Rotationseinrichtung (9) wie die erste Parallelgelenkeinrichtung gelegen, schwenkbar an einem Ende einer zweiten Verbindungseinrichtung (131) angelenkt ist;

einer zweiten Verbindungseinrichtung (131), die schwenkbar an einem Ende an dem ersten Ende des weiteren Gliedes und an dem anderen Ende an einem Punkt des einen Gliedes der Glieder (222) der ersten Parallelgelenkeinrichtung, der zwischen deren Enden liegt, angelenkt ist;

einer Gegengewichteinrichtung (130), die an dem zweiten Ende des weiteren Gliedes angebracht ist.

4. Szintillationskamergerät (200) nach Anspruch 3, bei dem:

die Rotationseinrichtung (9) ein kreisförmiges Ringglied hat, das drehbar an der Trägereinrichtung (2) über eine Vielzahl von Rollen (5, 8, 10, 11) angebracht ist;

die erste Parallelgelenkeinrichtung aus einem ersten Balken (122) und einem zweiten Balken (124) von gleichen Arbeitslängen a besteht;

die zweite Parallelgelenkeinrichtung aus einem Teil des ersten Balkens (122) und einem Teil eines dritten Balken (140) besteht, wobei die Arbeitslänge des anderen Teiles des dritten Balkens (140) a beträgt;

der erste Balken (222) schwenkbar über eine erste Balkenrotationsachse (121) an der Rotationseinrichtung (9) angelenkt ist und der dritte Balken (140) schwenkbar über eine dritte Balkenrotationsachse (128) an der Rotationseinrichtung (9) angelenkt ist;

der Abstand (b) zwischen der ersten Balkenrotationsachse (121) und der Rotationsachse (CM) gleich dem Abstand (b) zwischen der dritten Balkenrotationsachse (128) und der Rotationsachse (CM) ist; und

das Gewicht des Gegengewichtes (130) identisch zu demjenigen der Szintillationskameraeinrichtung (14) ist.

5. Szintillationskamergerät (300) mit:

einer Trägereinrichtung (2), die auf der Ebene eines Bodens festgelegt ist und sich in einer ersten Ebene senkrecht zur Bodenebene erstreckt;

einer Rotationseinrichtung (9), die eine Rotationsachse (CM) senkrecht zur ersten Ebene hat, wobei die Rotationseinrichtung drehbar in der ersten Ebene an der Trägereinrichtung (2) befestigt ist;

einer ersten Parallelgelenkeinrichtung mit:

parallelen Gliedern (322, 124), die schwenkbar an ihren ersten Enden an der Rotationseinrichtung (9) angelenkt sind, um schwenkbar in einer zweiten Ebene senkrecht zur ersten Eben zu sein, und die an ihren zweiten Enden schwenkbar an einer ersten Verbindungseinrichtung (127) angelenkt sind, wobei die Gelenkeinrichtung aus einem ersten Balken (322) und einem zweiten Balken (124) von gleichen Arbeitslängen a besteht, und wobei der erste Balken (322) schwenkbar über eine erste Balkenrotationsachse (121) an der Rotationseinrichtung (9) angelenkt ist;

einer ersten Verbindungseinrichtung (127), von der eine Ende schwenkbar an den parallelen Gliedern (322, 124) angelenkt ist und von der das andere Ende schwenkbar an der Szintillationskameraeinrichtung (14) angelenkt ist;

einer zweiten Gelenkeinrichtung mit:

einem dritten Balken (129) einer Arbeitslänge a, der schwenkbar an der Rotationseinrichtung (9) angelenkt ist, um in der zweiten Ebene schwenkbar zu sein, wobei ein Ende des dritten Balkens (129) schwenkbar über ein dritte Balkenrotationsachse (128) an der Rotationseinrichtung (9) angelenkt ist und

einer Gegengewichteinrichtung (130), die starr mit dem anderen Ende hiervon verbunden ist;

einer zweiten Verbindungseinrichtung mit einem Paar von Kettenrädern (150A, 150B), wobei eines dieser Kettenräder (150A) drehbar mit der ersten Balkenrotationsachse (121) verbunden und an dem ersten Balken festgelegt ist, und wobei das andere dieser Kettenräder (150B) drehbar mit der dritten Balkenrotationsachse (128) verbunden und an dem dritten Balken festgelegt ist, und mit einer Kette (152), die die Kettenräder (150A, 150B) verbindet.

**Revendications**

1. Une caméra à scintillation (100) comportant:

des moyens de support (2) qui sont fixés sur le plan d'un sol et qui s'étendent dans un premier plan perpendiculaire audit plan de sol;

des moyens de rotation (9) qui comportent un axe de rotation (CM) perpendiculaire audit premier plan, lesdits moyens de rotation étant assujettis auxdits moyens de support (2) de manière tournante dans ledit premier plan;

des premiers moyens à bielles parallèles comportant:

des éléments parallèles (122A, 124) portés de manière tournante à leur première extrémité par lesdits moyens de rotation (9) de manière à pouvoir pivoter dans un second plan perpendiculaire audit premier plan, et à leur seconde extrémité par des premiers moyens de liaison (127) dont une extrémité est portée de manière pivotante par lesdits éléments parallèles (122A, 124) et dont l'autre extrémité est portée de manière pivotante par ladite caméra à scintillation (14);

des seconds moyens à bielles parallèles comportant:

des éléments parallèles (122B, 129) portés de manière tournante à leur première extrémité par lesdits moyens de rotation (9) de manière à pouvoir tourner dans un second plan, et à leur seconde extrémité par des seconds moyens de liaison (131), lesdits éléments (122B, 129) s'étendant à partir du côté des moyens de rotation (9) qui est opposé auxdits premiers moyens à bielles parallèles;

un élément desdits premiers moyens à bielles parallèles et un élément des seconds moyens à bielles parallèles présentant un point commun de liaison aux moyens de rotation (9) et étant constitué par un élément commun (122), le point de liaison pour ledit élément commun (122) étant situé entre les points de liaison pour les autres éléments des premiers et seconds moyens à bielles parallèles;

des moyens à contrepoids (130) assujettis auxdits seconds moyens de liaison.

2. Une caméra à scintillation (100) selon la revendication 1, dans laquelle:

lesdits moyens de rotation (9) sont constitués par une bague circulaire qui est portée à pivotement par lesdits moyens supports (2) par l'intermédiaire d'une pluralité de rouleaux (5, 8, 10, 11);

lesdits premiers moyens à bielles parallèles sont constitués par une première moitié de poutre (122A) d'une première poutre (122) et par une seconde poutre (124) de mêmes longueurs utiles a;

lesdits seconds moyens à bielles parallèles sont constitués par une seconde moitié de poutre (122B) de ladite première poutre (122) et par une troisième poutre (129) de mêmes longueurs utiles a;

ladite première poutre (122) est portée de manière pivotante par lesdits moyens de rotation (9) par l'intermédiaire d'un arbre (121) de rotation de la première poutre, et ladite troisième poutre (129) est portée de manière pivotante par lesdits moyens de rotation (9) par l'intermédiaire d'un arbre (128) de rotation de la troisième poutre;

la distance (b) entre l'arbre (121) de rotation de la première poutre et l'axe de rotation (CM) est égale à la distance (b) entre l'arbre (128) de rotation de la troisième poutre et l'axe de rotation (CM); et

le poids du contrepoids (130) est identique à celui de ladite caméra à scintillation (14).

3. Une caméra de scintillation (200) comportant:

des moyens de support (2) qui sont fixés sur le plan d'un sol et s'étendent dans un premier plan perpendiculaire audit plan de sol;

des moyens de rotation (9) qui présentent un axe de rotation (CM) perpendiculaire audit premier plan, lesdits moyens de rotation étant assujettis aux moyens supports (2) de manière tournante dans ledit premier plan;

des premiers moyens à bielles parallèles comportant:

des éléments parallèles (222, 124) portés de manière tournante à leur première extrémité par lesdits moyens de rotation (9) de manière à pouvoir tourner dans un second plan perpendiculaire audit premier plan, et à leur seconde extrémité par des premiers moyens de liaison (127) dont une extrémité est portée de manière pivotante par lesdits éléments parallèles (222, 124) et dont l'autre extrémité est portée de manière pivotante par la caméra à scintillation (14);

des seconds moyens à bielles parallèles formés par:

une partie de l'un premier des éléments (222) desdits moyens à bielles parallèles et par une partie d'un autre élément (140) porté à pivotement en un poitn situé entre ses extrémités par les moyens de rotation (9) de manière à pouvoir tourner dans ledit second plan, une première extrémité dudit autre élément, située du même côté des éléments de rotation (9) que les premiers moyens à bielles parallèles, étant portée par une première extrémité de seconds moyens de liaison (131);

les seconds moyens de liaison (131) étant portés de manière pivotante à une extrémité par ladite première extrémité dudit autre élément, et à son autre extrémité en un point dudit premier élément (122) desdits premiers moyens à bielles parallèles situé entre ses extrémités;

des moyens à contrepoids (130) assujettis à la seconde extrémité dudit autre élément.

4. Une caméra à scintillation (200) selon la revendication 3, dans laquelle:

· lesdits moyens de rotation (9) sont constitués par une bague circulaire qui est portée à pivotement par lesdits moyens supports (2) par l'intermédiaire d'une pluralité de rouleaux (5, 8, 10, 11);

lesdits premiers moyens à bielles parallèles sont constitués par une première poutre (222) et une seconde poutre (124) de même longueur utile a;

lesdits seconds moyens à bielles parallèles sont constitués par une partie de ladite première poutre (222) et une partie d'une troisième poutre (140), la longueur utile de l'autre partie de la troisième poutre (140) étant égale à a;

ladite première poutre (222) est portée à pivotement par lesdits moyens de rotation (9) par l'intermédiaire d'un arbre (121) de rotation de la première poutre, et ladite troisième poutre (140) est portée de manière pivotante par lesdits moyens de rotation (9) par l'intermédiaire d'un arbre (128) de rotation de la troisième poutre;

la distance (b) entre l'arbre (121) de rotation de la première poutre et l'axe de rotation (CM) est égale à la distance (b) entre l'arbre (128) de rotation de la troisième poutre et l'axe de rotation (CM); et

le poids du contrepoids (130) est égal à celui de la caméra à scintillation (14).

5. Une caméra à scintillation (300) comportant:

des moyens de support (2) qui sont fixés sur le plan d'un sol et qui s'étendent dans un premier plan perpendiculaire audit plan de sol;

des moyens de rotation (9) qui présentent un axe de rotation (CM) perpendiculaire audit premier plan, lesdits moyens de rotation étant assujettis auxdits moyens supports (2) de manière tournante dans ledit premier plan;

des premiers moyens à bielles parallèles comportant:

des éléments parallèles (322, 124) portés de manière pivotante à leur première extrémité par lesdits moyens de rotation (9) de manière à pouvoir tourner dans un plan perpendiculaire audit premier plan, et à l'autre extrémité par des premiers moyens de liaison (127), lesdits moyens à bielles étant constitués par une première poutre (322) et par une seconde poutre (124) présentant les mêmes longueurs utiles a, ladite première poutre (322) étant portée par lesdits moyens de rotation (9) de manière pivotante par l'intermédiaire d'un arbre (121) de rotation de la première poutre;

des premiers moyens de liaison (127) dont une extrémité est portée de manière pivotante par lesdits éléments parallèles (322, 124) et dont l'autre extrémité est portée de manière pivotante par la caméra à scintillation (14);

des seconds moyens à bielles comportant:

une troisième poutre (129) de longueur utile a, qui est portée de manière pivotante par les moyens de rotation (9) de manière à pouvoir pivoter dans ledit second plan, une extrémité de ladite troisième poutre (129) étant portée de manière pivotante par lesdits moyens de rotation par l'intermédiaire d'un arbre (128) de rotation de la troisième poutre, et des moyens à contrepoids (130) étant rigidement reliés à son autre extrémité;

des seconds moyens de liaison comportant une paire de roues à chaîne (150A, 150B), l'une (150A) desdites roues à chaîne étant reliée à rotation à l'arbre (121) de rotation de la première poutre et étant fixée à ladite première poutre, et l'autre (150B) desdites roues à chaîne étant reliée à rotation à l'arbre (128) de rotation de la troisième poutre et étant fixée à ladite troisième poutre, et une chaîne (152) reliant les roues à chaîne (150A, 150B).

# F I G. 1A

# F I G. 1B

# F I G. 2A

# F I G. 2B

# F I G. 3

# F I G. 4

# F I G. 5

4